# EUROPEAN PATENT SPECIFICATION

(11) **EP 4 217 189 B1**
(45) Date of publication and mention of the grant of the patent: **14.08.2024**
(21) Application number: 22800004.8
(22) Date of filing: 29.08.2022
(51) Int. Cl.: B32B 27/08, B32B 27/32, B32B 27/30

(54) **SINGLE-USE BIOPROCESSING BAG FOR GROWING CELL MEDIA, AND MULTILAYER FILM**
EINWEG-BIOVERARBEITUNGSBEUTEL ZUM ZÜCHTEN VON ZELLMEDIEN UND MEHRSCHICHTIGE FOLIE
SAC DE BIOTRAITEMENT À USAGE UNIQUE POUR LA CULTURE DE MILIEUX CELLULAIRES ET FILM MULTICOUCHE

(30) Priority: 30.08.2021 US 202163238675 P
(43) Date of publication of application: 02.08.2023
(73) Proprietor: Tekni-Plex, Inc., Wayne, PA 19087 (US)
(72) Inventor: VAN LANDEGHEM, Robin, 9820 Deinze (BE)
(74) Representative: Page White Farrer
(86) International application number: PCT/US2022/041832
(87) International publication number: WO 2023/034188

(56) References cited:
- US-A1- 2017 175 066

## Description

### Field of the Invention

The present invention relates to a single-use bioprocessing or bioreactor bag (e.g., for growing cell media), and to multilayer films for producing such bags.

### Background

Metal and glass reactor systems are well known for conducting various types of reactions in the bio-pharmaceutical industry, including for example, synthesis, cell growth and other bio-reactions. However, such systems are best suited for large scale production, as opposed to producing small quantities or batches. For the latter, a single-use plastic bag is better suited in terms of set-up time, maintenance, cost and adaptability (to product changes).

Metal or glass reactor systems do have certain advantages, a major one being that they are inert. In contrast, a plastic container for growing cell media may leach components (of the bag) into the media during reaction, storage or transport. Such leached components may contaminate the media or adversely influence the reaction rate. For example, it would be desirable to avoid the use of certain heat stabilizers that are commonly used in the polymer industry, such as the plastics heat stabilizer tris(2,4-di-tert-butylphenyl)phosphite and its breakdown product bis(2,4-di-tert-butylphenyl)phosphate, which has been found to have a strong negative effect on cell growth rate.

The ideal bag must satisfy a number of requirements relating to strength, flexibility, ease of manufacture (including both multilayer film production and sealing the film to form a bag), gas and moisture barrier properties, resistance to sterilization (without degradation of properties), optical properties and appearance. Avoiding use of a heat stabilizer such as that noted above may have a deleterious effect on the film and/or bag mechanical/barrier/optical properties and thus while solving the leaching problem, creates other performance and/or manufacturing problems.

It would be desirable to provide a single-use plastic film bag that can satisfy the above noted requirements and that avoids the use of phosphite heat stabilizers.

US-A-2017/175066 discloses a single-use bag for biological materials and processing, including use in a disposable cell culture bioreactor, which includes a collapsible a body having a first end and an opposing second end bounding a compartment. The collapsible body comprises at least one flexible sheet having an interior surface and an exterior surface; wherein the interior surface layer comprises low density polyethylene having a melt index from 0.9 to 2.0 g/10 min with a weight average molecular weight (Mw) greater than 3.321 × 10⁻²² Kg (200,000 Da), and an Mz greater than 2.49 × 10⁻²⁰ Kg (1,500,000 Da).

### Summary

In a first aspect, the present invention provides a multilayer bioreactor bag, made from a multilayer film, according to claim 1. Optional or preferred features are defined in claims 2 to 10.

In a second aspect, the present invention provides a multilayer film according to claim 11. Optional or preferred features are defined in claims 12 to 15.

The preferred embodiments of the present invention are directed to a single-use plastic bioprocessing bag (e.g., for growing cell media and other bioreactions), and to a multilayer film useful in producing such bags. The multilayer film and bag of the preferred embodiments avoid the problems of leaching undesirable components into the cell media, while promoting high cell growth rates and providing a range of desirable bag properties that facilitate both use and manufacture of the bag.

In one embodiment, a single-use bioprocessing bag for growing cell media is formed from a multilayer film including inner (adjacent the cell media) and outer (adjacent the surrounding environment) exterior layers of a low density polyethylene polymer, and intermediate layers of: a) an ethylene alpha-olefin copolymer (such as ethylene based octene-1 elastomer for flexibility and structural support), b) an intermediate gas barrier layer (such as ethylene vinyl alcohol copolymer), and c) tie layers between the various layers (such as a tie layer of an ethylene alpha-olefin copolymer and polyolefin copolymer grafted with maleic anhydride).

Prior multilayer films for storage bags have used ethylene alpha-olefin copolymers as the exterior layers. However, they have a problem with processability and handling due to the tacky nature of the material. In addition, they have required the use of heat stabilizers which often have other deleterious effects, such as leaching and/or a reduction of cell growth rate.

The present invention solves these problems by separating out a polyolefin layer having a certain density range, from an ethylene-alpha olefin intermediate layer having a higher density range (than the polyethylene). This avoids problems with poor handling, leaching and reduced cell growth of the prior art film bags, while providing a favorable combination of bag properties including strength, flexibility, barrier, optical/transparency and appearance.

These and other advantages of the present invention are disclosed in the following detailed description and drawings.

### Brief Description of the Drawings

Fig. 1 is a schematic perspective view of a single-use bioprocessing (bioreactor) bag (20) according to one embodiment of the invention;
Fig. 2 is an exemplary view of a processing facility (5) utilizing plastic bioreactor bags (6) for growing cell media such as that provided by the present invention; each bioreactor bag has associated tubing (7), e.g., for connecting the bag to input and output fluids;
Fig. 3 is a cross-sectional schematic view of a section of multilayer film (10) for use in a bioreactor bag, the film having six (6) layers in serial order as shown, according to one embodiment of the invention;
Fig. 4 is a chart listing the layer structure, materials and various material properties of the 6-layer film shown in Fig. 3;
Fig. 5A is a chart listing various testing methods used to characterize the properties of the bioreactor bag and 6-layer film of the present embodiment, while Figs. 5B-5E illustrate various test data and measurement techniques; Fig. 5B includes a chart with test results and graphs of mechanical properties for the multilayer film; Fig. 5C includes a chart with test results and an illustration of a test apparatus for puncture resistance testing; Fig. 5D includes a chart with test results for heat seal testing; Fig. 5E includes a chart with test results for optical property testing,

### Detailed Description

### A. Resins

The resins of the respective layers in the multilayered film, bioreactor bag and method of producing the multilayered film and bag according to various embodiments of the present invention are described herein.

### Low density polyethylene

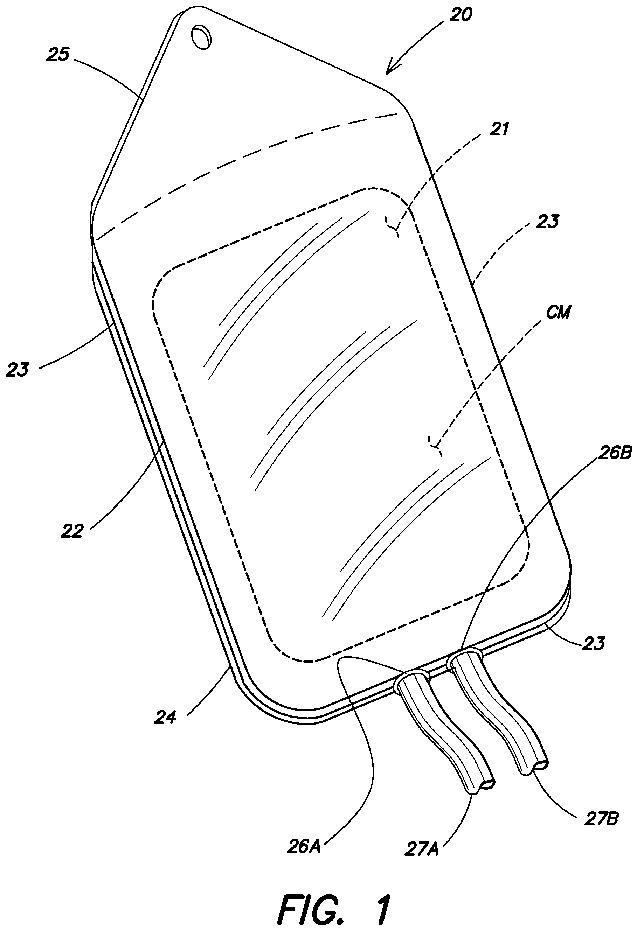

The low density polyethylene LDPE used herein for the innermost and outermost exterior layers ((11) and (16) respectively of multilayer film (10) forming bag (20, 6) in Figs. 1-3) has a density in a range of 910 kg/m³ to 940 kg/m³ (kilograms per cubic meter) (0.91-0.94 g/cc (grams per cubic centimeter)), according to ASTM 1505, and is preferably of a high level of purity and without additives. The LDPE is suitable for lamination and extrusion of multilayer films and contributes to increased tensile strength properties in the stated density range (Young's modulus, e.g. 190 MPa). In contrast, the ethylene alpha-olefin copolymer (for the interior layer(s) (12) of the multilayer film (10)) as described below in the stated density range has a much lower modulus (e.g., 24 MPa) to provide higher flexibility, (particularly in view of its greater percentage of the total multilayer film thickness). The LDPE exterior layers (11, 16) provide a good combination of strength and transparency, while being sufficiently flexible to resist tearing or puncture. The interior alpha-olefin copolymer layers (12) provide heat resistance as described further below.

One example of a suitable LDPE polymer is Sabic PCG02, a low density polyethylene (921 kg/m³ (.921 g/cc according to ASTM D1505)) available from Sabic Innovative Plastics BV of the Netherlands (www.sabic.com), produced in a high pressure tubular reactor, that is free of bis-(2, 4-di-tert-butylphenol)pentaerythritol diphosphate. It complies with the monographs of the European Pharmacopoeia and the United States Pharmacopoeia. The resin has a typical melt flow rate of 1.9 dg/min. according to ISO 1133 (at 190 degrees C and 2.16 kg). Optical properties include a typical gloss (45 degrees) of 53%, ASTM D 2457, and typical haze of 10%, ASTM D 1003. As a film the LDPE has a typical value of impact strength of 20 kJ/m, ASTM D 4272, and tear strength of 25 kN/m in the transverse direction and 70 kN/m in the machine direction, ISO 6383-2.

### Ethylene alpha-olefin copolymer elastomer

The ethylene alpha-olefin copolymer elastomer includes, for example, ethylene monomer copolymerized with an alpha-olefin having 3 to 12 carbon atoms, such as propylene, 1-butene, 1-pentene, 1-hexene, 4-methyl-1-pentene, 1-heptene, 1-octene, 1-nonene, 1-decene, 1-undecene, 1-dodecene or the like, in which the molecules of the copolymers comprise polymer chains with relatively few side chain branches arising from the alpha-olefin which was reacted with ethylene.

It is preferred that the ethylene alpha-olefin copolymer elastomer has a density in a range of 870 to 910 kg/m³ (0.87 to 0.91 g/cm³). The melt flow rate MFR is preferably in a range of 1 to 2 g/10 minutes (190°C) per ISO 1133 and the melting point is preferably from 115 to 125°C.

A suitable copolymer elastomer is ethylene based octene-1 elastomer, such as Bormed PL8830-PH available from Borealis AG Vienna Austria, www.borealisgroup.com. PL8830-PH is produced in a solution polymerization process using a metallocene catalyst, and contains a low amount of processing stabilizers, and a very low level of antioxidant. The resin has a typical density of 883 kg/m³ (.883 g/cc), per ISO 1183-1 Method A, and a typical melt flow index of 1.1 g/10 minutes (190°C /2, 16 kg) per ISO 1133.

### Polyolefin Copolymer grafted with maleic anhydride

The polyolefin copolymer grafted with maleic anhydride is provided in the tie layers (13, 15), providing excellent adhesion to the adjacent layers (ethylene alpha-olefin copolymer and/or LDPE). One example of a suitable resin is Bynel 46E1052 available from Dow Chemical, Midland Ml.

### Polyethylene co-vinyl alcohol (EVOH)

The polyethylene co-vinyl alcohol (EVOH) copolymer referred to herein for barrier layer (14) can be obtained from any commercial source. For example, an extrusion grade EVOH is available under the name EVAL^{™} from Kuraray Co. Ltd. of Japan. The ethylene vinyl alcohol copolymer employed herein can have a vinyl alcohol content ranging from about 40 to about 85 mole percent (mol%), and preferable, from about 50 to about 75 mol%.

The EVOH layer provides both a gas barrier and moisture barrier properties to the multilayer film. Other barrier materials can be used depending on the desired application, such as polyamide, polyester and polyvinyl dichloride. Preferably the oxygen barrier polymer has an oxygen permeability of less than 500 cc O2/meter-squared * day* atmosphere (tested at 1 mil thickness and at 25 degrees C per ASTM D3985, and more preferably less than 100.

### B. Film Layers

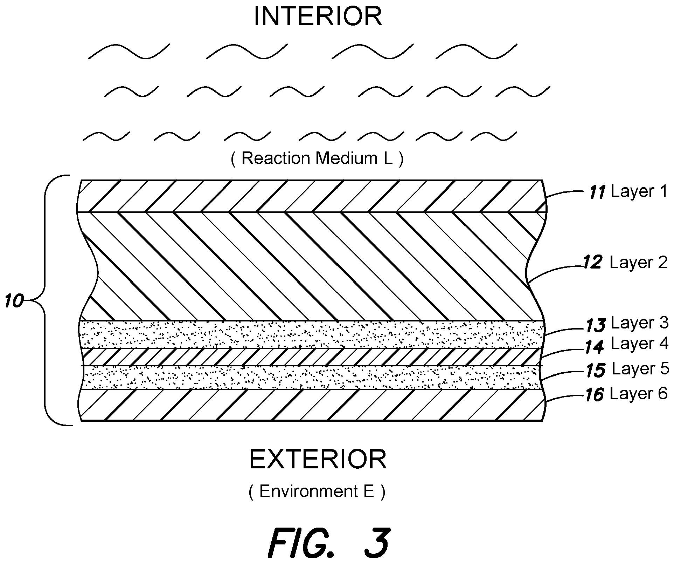

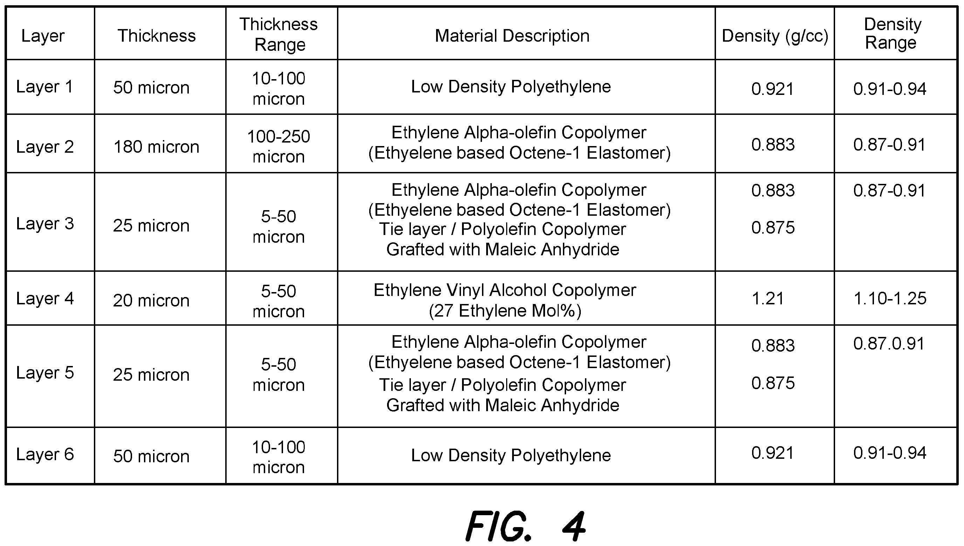

As an illustrative example, a multilayer film (10) depicted in Fig. 3 and described in Fig. 4 has two exterior layers (11, 16) and four intermediate (interior) layers (12, 13, 14, 15). Layer 1 is the inner layer (11) of the bag; it is made from the LDPE and is in contact with the reaction medium L in the bag. Layer 6 is the outer layer (16) of the bag (in contact with the environment E); it is also LDPE and has the same or different thickness as Layer 1. The composition and thickness of the exterior layers (11, 16) is chosen to enhance the certain properties of the multilayer film such as strength, rigidity, puncture resistance, gas permeability, gamma radiation resistance, heat seal and heat processing resistance, and/or the ultraviolet (UV) resistance.

In between the exterior inner Layer 1 (11) and the exterior outer Layer 6 (16), are four intermediate Layers 2-5 (12, 13, 14, 15 respectively). Fig 3 shows a six-layered film having in serial order: Layer 1 of LDPE; Layer 2 (adjacent Layer 1) of ethylene alpha-olefin copolymer, a tie Layer 3 (adjacent Layer 2), Layer 4 (adjacent Layer 3) of polyethylene co-vinyl alcohol (EVOH) as a gas barrier layer, another tie Layer 5 (adjacent Layer 4) and finally exterior Layer 6 (adjacent Layer 5). The tie Layers 3 and 5 improve the adhesion between the functional layers and prevent film failure through delamination.

In another embodiment, the EVOH Layer 3 can be disposed closer to the exterior inner Layer 1 (closer to the interior of the bag).

### C. Methods and Thickness

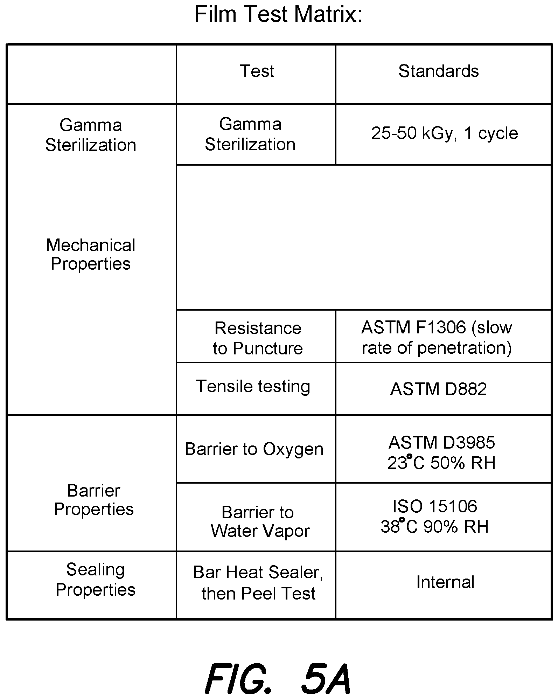

Fig. 4 is a chart listing the preferred materials, thicknesses, and properties of the various layers ((11)-(16) of multilayer film (10)) according to one embodiment of the present invention, as discussed further below. This embodiment is referred to as A in the subsequent test protocols of Figs. 5A-5E.

Various methods can be used to produce the multi-layered film of the present invention, for example, a water-cooling or air-cooling coextrusion inflation method, a coextrusion T-die method, a dry lamination method and an extrusion lamination method. In view of desired performance characteristics, particularly transparency, economy and sanitation, a water-cooling coextrusion inflation method and a coextrusion T-die method are preferably used.

The method is caried out at the temperature at which the resins of the respective layers become or are molten. When the temperature is raised too high, heat deterioration may occur in all or a portion of the resin, and deterioration in performance may result. Accordingly, the temperature for production of the multilayered film is usually set within a range from 150 to 200°C.

The total thickness of the multilayer film (10) of the present invention is generally in a range from 250 to 400 µm, and preferably from 300 to 400 µm, but can be varied appropriately depending on the desired use. Fig. 4 lists a preferred thickness range for the various layers as follows: Layer 1 (LDPE), 10-100 micrometers; Layer 2 (ethylene alpha-copolymer), 100-250 micrometers; Layer 3 (tie), 5-50 micrometers; Layer 4 (EVOH gas barrier), 5-50 micrometers; Layer 5 (tie), 5-50 micrometers; and Layer 6 (LDPE), 10-100 micrometers. This distribution of thicknesses and materials and order of materials has been found to provide a superior balance of performance in terms of mechanical properties (strength and flexibility), processability (film formation and handling), and cell growth rate, while avoiding the use of process stabilizers that may deleteriously affect the cell growth rate. The ratio of the exterior LDPE film thicknesses of each exterior Layer 1 and 6 (range of 10-100 with example 50 micrometers) versus the ethylene alpha-olefin copolymer Layer 2 (range of 100-250 with example 180 micrometers), is a ratio of between 1:2.5 and 1:10, with the much thicker alpha-olefin copolymer layer providing heat resistance (to prevent degradation of performance properties due to multilayer film formation, heat sealing and gamma sterilization), while the much thinner exterior layers of LDPE provide a desired non-tacky surface for improved film and bag handling. Fig. 4 also lists the preferred density ranges for each of Layers 1-6.

### D. Evaluation and Performance Tests

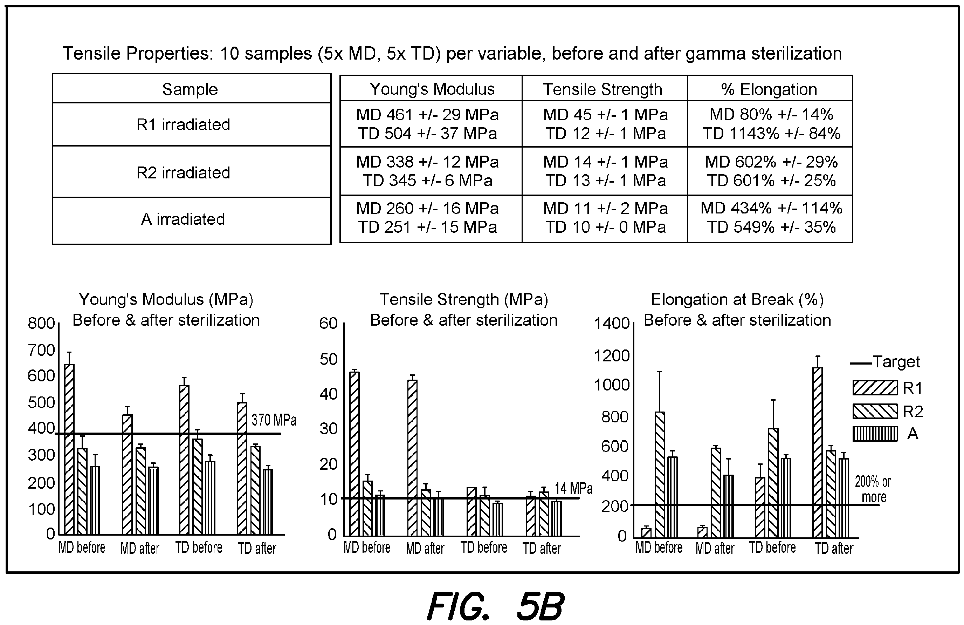

Fig. 5A is a list of various properties and testing standards for evaluating samples of the film materials used according to the described embodiment, including: Gamma Sterilization; Resistance to Puncture, Tensile Testing, Barrier to Oxygen, Barrier to Water, and Heat Sealing. Figs. 5B-5E show the results and measuring apparatus for select tests. The present embodiment is denoted by sample A, compared to two reference sample R1 and R2 that provide a basis of comparison.

Young's Modulus is a mechanical property of the material that represents the tensile stiffness of a solid material (e.g., film). It is measured as a proportionality function of the tensile stress/tensile strain (FL)/(A * change in L), where the force F is the applied force, L is the initial length, A is the square area, and the resulting Young's Modulus is in Pascals (Pa). The higher the modulus, the more stress is needed to create the same amount of strain; an idealized rigid body would have an infinite Young's modulus, while a very soft material such as a fluid, could deform without force and have a zero Young's modulus. See JIS-K-7105 and JIS-K-7136.

Tensile strength and tensile elongation express the maximum force the film material can withstand when it is pulled before breaking; the ultimate tensile strength is the highest point on the stress-strain curve and the tensile elongation is the amount it stretches. They can be measured (according to ASTM-D-882) using a tensile testing machine and the values provided in units of MPas (mega Pascals).

Fig. 5B sets forth the tensile testing results, including Young's modulus, Tensile Strength and % Elongation for the two reference samples R1 and R2, and for the embodiment of the present invention A. The samples were tested both before and after gamma sterilization, in both the machine direction MD and the transverse direction TD. In the bar graphs, the results are shown in groups of three with the left bar being reference R1, the middle bar R2, and the right bar A. The horizontal line across each set of bar graphs represents the desired target for this one embodiment. Other targets may be selected depending upon the particular use application. In the present embodiment, the invention A was shown to provide consistent mechanical properties that are not significantly adversely affected by the gamma sterilization.

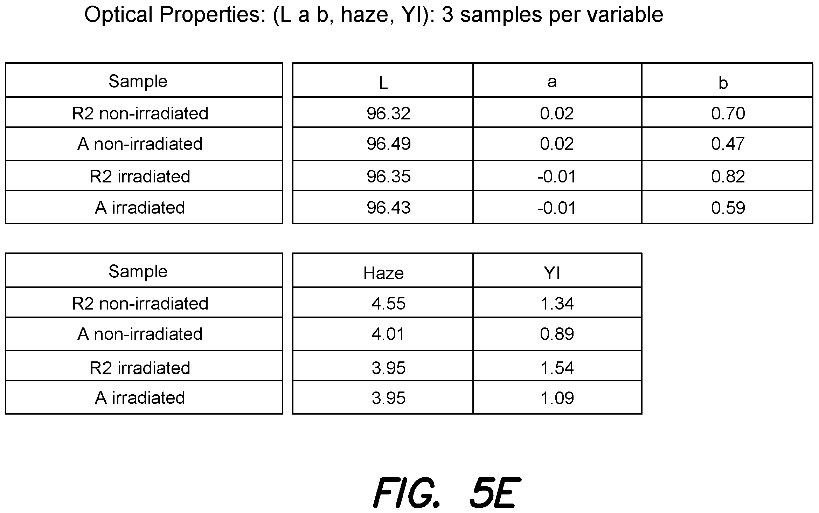

Haze is an index related to transparency of the film and represents the amount of haziness (cloudiness); it is obtained from the ratio of diffuse transmitted light to the total light transmitted; it is affected by the amount of surface roughness. Fig. 5E lists various optical properties: color indices L, a and b; Haze, as a %; and yellowness index Y1, showing the reference materials R1 and R2 and current embodiment of the invention A, prior to (nonirradiated) and after being subject to gamma sterilization (irradiated).

Heat Resistance (effect of gamma sterilization): The films were gamma sterilized at a dose of 50kGy, before being assembled into a bag. The properties were measured and compared to those prior to gamma sterilization.

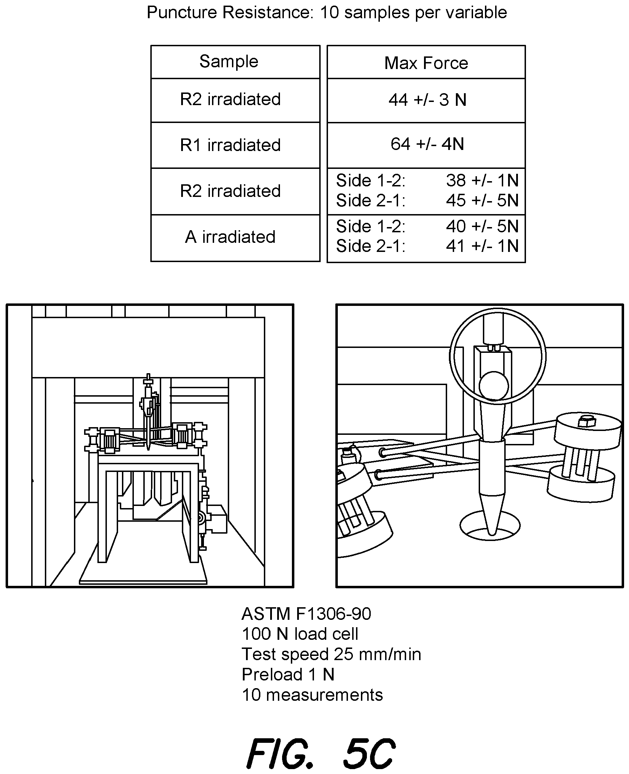

Puncture Resistance was measured with the load cell equipment shown in Fig. 5C according to ASTM F1306-90, with a 100 N (Newtons) load cell, test speed of 0.000417 m/s (meters per second) (25 mm/min (millimeters per minute)), and preload of 1 N, for 10 measurements. Fig. 5C lists the Maximum Force the sample could withstand (without generating a tear or puncture), showing the reference materials R1 and R2 and current embodiment of the invention A, after being irradiated by gamma sterilization.

Flexibility: GelboFlex was conducted according to conditions C, D, E as described by ASTM F392, and no pinholes were detected.

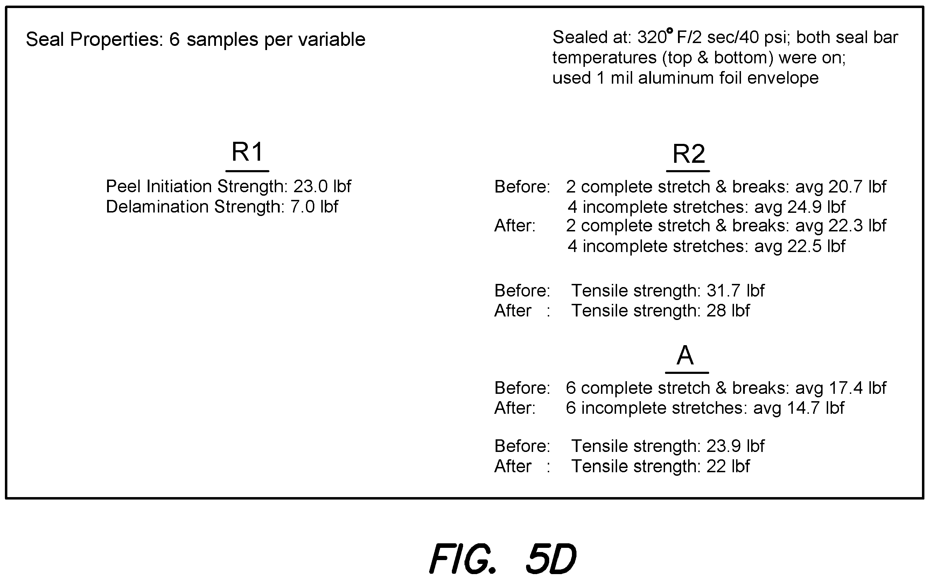

The heat sealing properties and testing protocol are shown in Fig. 5D, with measurements of peel initiation strength and delamination strength provided for the reference samples R1 and R2, and for the sample embodiment of the invention A. Two exterior layers of two multilayer films were heat sealed at 160 degrees Celsius/2 seconds/275.79kPa (320 degrees Fahrenheit/2 seconds/40 psi), and the force measured to initiate peeling between the two exterior layers of the two multilayer films, along with the delamination strength.

### E. Bag

The multilayer films can be used to manufacture single-use system SUS articles. Examples of preferred articles are films and/or bags, especially for bioprocessing applications and/or pharmaceutical applications. The films are particularly well suited to make disposable, sterile bioreactor bags. Such bags may be used for storage and/or for executing cell cultures and (bio)chemical reactions. The bioreactor bags can be made in a variety of sizes to suit different process steps and scale-up stages. Bag volumes may range from about 0.1L to about 5.0L for laboratory settings and up to about 10,000L for pilot, pre-production, and production scales.

The bioreactor bag consists of the multilayer film components listed above and preferably no phosphite antioxidant compound/component or degradation product is present.

In certain embodiments, the bioreactor bag may be an inflatable bag (20) having an enclosed central chamber (21) for holding a cell media CM as shown in Fig. 1. The bag comprises a top sheet (22) of multilayer polymer material and a bottom sheet (24) of multilayer polymer material coupled along at least one edge of the top sheet to form a sealed bag, wherein the multilayer polymer material is formed with the layers (e.g., multilayer film (10) of Fig. 3) as previously described. In this example the two rectangular sheets (22, 24) are heat sealed along all four perimeters edges (23) of the generally rectangular shaped bag (20) to form the central enclosed chamber (21). The top edge includes a handle opening (25), and the bottom edge has two openings or ports (26A) and (26B), through which two tubes (27A) and (27B) are respectively inserted to allow liquids and/or gases to enter and/or exit the central bag chamber (21). The top sheet and the bottom sheet may be formed from separate sheets as shown, or may be formed from a contiguous sheet folded at least partially upon itself. Other configurations of material may be used. The bioreactor bag may further comprise additional sheets or films or gaseous spacer layers

Additionally, the bioreactor bag may include additional layers, such as structural and tie layers that contribute to the overall structural integrity of the bag, improve adherence among the film layers, and prevent delamination. Useful materials for additional structural layers include anhydride-modified polyethylene, ethylene/unsaturated acid copolymer, ethylene/unsaturated ester copolymer, anhydride-modified polyolefin, polyurethane, and mixtures thereof. Preferably, the structural layer includes anhydride-modified polyethylene. One example of a commercially available anhydride-modified polyethylene is Bynel^{™} 4157 from the E.I. du Pont de Nemours and Company of Wilmington, Delaware (DuPont).

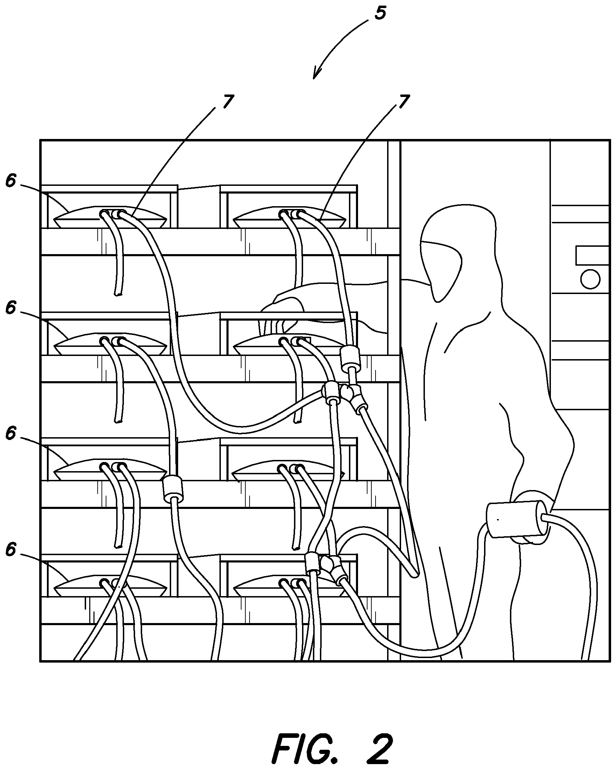

Fig. 2 illustrates one use of a multilayer bioreactor bag of the present invention in a bioprocessing facility (5), for use with commercial rocking-motion bioreactor chambers. Each single-use cell culture bag (6) is provided in 2L-50L configurations and features bag ports for connecting to associated tubing (7). The rocking-motion of the bioreactor platform creates effective, non-invasive mixing of the cell culture media with efficient gas transfer. Rocking-motion prevents cells from settling at the bottom of the culture bag while generating large amounts of recombinant protein in both mammalian and insect cell lines. The closed-system eliminates the risk of cross-contamination. This single-use cell culture bag is well-suited for basic research to large-scale biopharmaceutical manufacturing applications.

These and other embodiments of the invention will be apparent to the skilled person and the invention is not limited to the foregoing examples.

## Claims

1. A multilayer bioreactor bag (20) made from a multilayer film (10) comprising:
inner and outer exterior surface layers (11, 16) on opposing faces of the multilayer film (10) consisting essentially of a low density polyethylene having a high level of purity and without additives and having a density in a range of 910 to 940 kg/m³ (0.91 to 0.94 g/cc) to provide non-tacky exterior surface layers;
interior layers disposed between the exterior surface layers comprising a gas barrier layer (14) and a structural layer (12) comprising an ethylene alpha-olefin elastomer having a density in a range of 870 to 910 kg/m³ (0.87-0.91 g/cc) to provide a flexible support to the multilayer film.

2. The multilayer bioreactor bag of claim 1, wherein the multilayer film further comprises one or more tie layers (13, 15) to bond the interior layers to one or more of the ethylene alpha-olefin elastomer structural layer (12) and surface layers (11, 16).

3. The multilayer bioreactor bag of claim 2, wherein the one or more tie layers (13, 15) comprise a mixture of an ethylene alpha-olefin elastomer and a polyolefin copolymer grafted with maleic anhydride.

4. The multilayer bioreactor bag according to any of the preceding claims, wherein the multilayer film comprises six layers (Layers 1-6) in serial order as listed below:
Layer 1: the inner exterior surface layer (11);
Layer 2: a first ethylene alpha-olefin elastomer structural layer (12);
Layer 3: a first tie layer (13);
Layer 4: the gas barrier layer (14);
Layer 5: a second tie layer (15); and
Layer 6: the outer exterior surface layer (16);
optionally wherein the thickness ranges of the six layers are:
Layer 1 is in a thickness range of 10-100 micrometers;
Layer 2 is in a thickness range of 100-250 micrometers;
Layer 3 is in a thickness range of 5-50 micrometers;
Layer 4 is in a thickness range of 5-50 micrometers;
Layer 5 is in a thickness range of 5-50 micrometers; and
Layer 6 is in a thickness range of 10-100 micrometers;
further optionally wherein the density ranges of the six layers are:
Layer 1 is in a density range of 910 to 940 kg/m³ (0.91-0.94 g/cc);
Layer 2 is in a density range of 870 to 910 kg/m³ (0.87-0.91 g/cc);
Layer 3 is in a density range of 870 to 910 kg/m³ (0.87-0.91 g/cc);
Layer 4 is in a density range of 110 to 125 kg/m³ (1.10-1.25 g/cc);
Layer 5 is in a density range of 870 to 910 kg/m³ (0.87-0.91 g/cc); and
Layer 6 is in a density range of 910 to 940 kg/m³ (0.91-0.94 g/cc).

5. The multilayer bioreactor bag according to any of the preceding claims, wherein the gas barrier layer (14) is EVOH.

6. The multilayer bioreactor bag of claim 1, comprising an inflatable bag (20) having an enclosed central chamber (21) for holding a cell or bioreactor liquid media, the inflatable bag (20) comprising a top sheet (22) of multilayer polymer material and a bottom sheet (24) of multilayer polymer material, wherein the multilayer polymer material comprises the multilayer film (10).

7. The multilayer bioreactor bag of claim 6, wherein the top sheet (22) and the bottom sheet (24) are heat sealed along perimeter edges to form the central chamber (21), optionally wherein a handle opening (25) is provided in a top perimeter edge of the bag (20); and port openings for receiving tubes (27) are provided in a bottom perimeter edge of the bag (20).

8. The multilayer bioreactor bag of any of the preceding claims 1 to 7, wherein the interior layers comprise one or more of anhydride-modified polyethylene, ethylene/unsaturated acid copolymer, ethylene/unsaturated ester copolymer, anhydride-modified polyolefin, polyurethane, and mixtures thereof.

9. The multilayer bioreactor bag of claim 1 comprising a single use bioprocessing bag for growing cell media wherein:
the ethylene alpha-olefin elastomer is ethylene based octene-1 elastomer; and
the gas barrier layer is ethylene vinyl alcohol copolymer.

10. The multilayer bioreactor bag of any of the preceding claims 1-9, wherein the ratio of layer thickness of each of the exterior surface layers (11, 16) to layer thickness of structural layer (12) is between 1:2.5 and 1:10.

11. A multilayer film (10) comprising:
inner and outer exterior surface layers (11, 16) on opposing faces of the multilayer film (10) consisting essentially of a low density polyethylene having a high level of purity and without additives and having a density in a range of 910 to 940 kg/m³ (0.91 to 0.94 g/cc) to provide non-tacky exterior surface layers;
interior layers disposed between the exterior surface layers comprising a gas barrier layer (14) and a structural layer (12) comprising an ethylene alpha-olefin elastomer having a density in a range of 870 to 910 kg/m³ (0.87-0.91 g/cc) to provide a flexible support to the multilayer film; and
one or more tie layers (13, 15) to bond the interior layers to one or more of the ethylene alpha-olefin elastomer structural layer (12) and surface layers (11, 16).

12. The multilayer film of claim 11, wherein the one or more tie layers (13, 15) comprise a mixture of an ethylene alpha-olefin elastomer and a polyolefin copolymer grafted with maleic anhydride.

13. The multilayer film according to claim 11 or claim 12, wherein the multilayer film comprises six layers (Layers 1-6) in serial order as listed below:
Layer 1: the inner exterior surface layer (11);
Layer 2: a first ethylene alpha-olefin elastomer structural layer (12);
Layer 3: a first tie layer (13);
Layer 4: the gas barrier layer (14);
Layer 5: a second tie layer (15); and
Layer 6: the outer exterior surface layer (16);
optionally wherein the thickness ranges of the six layers are:
Layer 1 is in a thickness range of 10-100 micrometers;
Layer 2 is in a thickness range of 100-250 micrometers;
Layer 3 is in a thickness range of 5-50 micrometers;
Layer 4 is in a thickness range of 5-50 micrometers;
Layer 5 is in a thickness range of 5-50 micrometers; and
Layer 6 is in a thickness range of 10-100 micrometers;
further optionally wherein the density ranges of the six layers are:
Layer 1 is in a density range of 910 to 940 kg/m³ (0.91-0.94 g/cc);
Layer 2 is in a density range of 870 to 910 kg/m³ (0.87-0.91 g/cc);
Layer 3 is in a density range of 870 to 910 kg/m³ (0.87-0.91 g/cc);
Layer 4 is in a density range of 110 to 125 kg/m³ (1.10-1.25 g/cc);
Layer 5 is in a density range of 870 to 910 kg/m³ (0.87-0.91 g/cc); and
Layer 6 is in a density range of 910 to 940 kg/m³ (0.91-0.94 g/cc).

14. The multilayer film according to any of the preceding claims 11 to 13, wherein the gas barrier layer (14) is EVOH.

15. The multilayer film according to any of the preceding claims 11 to 14, wherein the interior layers comprise one or more of anhydride-modified polyethylene, ethylene/unsaturated acid copolymer, ethylene/unsaturated ester copolymer, anhydride-modified polyolefin, polyurethane, and mixtures thereof.

## Patentansprüche

1. Mehrschichtiger Bioreaktorbeutel (20), hergestellt aus einer Mehrschichtfolie (10), umfassend:
innere und äußere externe Oberflächenschichten (11, 16) auf gegenüberliegenden Seiten der Mehrschichtfolie (10), die im Wesentlichen aus einem Polyethylen niedriger Dichte mit hohem Reinheitsgrad und ohne Zusatzstoffe bestehen und eine Dichte in einem Bereich von 910 bis 940 kg/m3 (0,91 bis 0,94 g/cm³) aufweisen, um nichtklebrige externe Oberflächenschichten zu erzeugen;
interne Schichten, die zwischen den externen Oberflächenschichten angeordnet sind, die eine Gassperrschicht (14) und eine Strukturschicht (12) umfassen, die ein Ethylen-Alpha-Olefin-Elastomer mit einer Dichte in einem Bereich von 870 bis 910 kg/m3 (0,87-0,91 g/cm³) umfasst, um einen flexiblen Träger für die Mehrschichtfolie bereitzustellen.

2. Mehrschichtiger Bioreaktorbeutel nach Anspruch 1, wobei die Mehrschichtfolie ferner eine oder mehrere Verbindungsschichten (13, 15) umfasst, um die internen Schichten mit einer oder mehreren der Ethylen-Alpha-Olefin-Elastomer-Strukturschichten (12) und der Oberflächenschichten (11, 16) zu verbinden.

3. Mehrschichtiger Bioreaktorbeutel nach Anspruch 2, wobei die eine oder mehreren Verbindungsschichten (13, 15) ein Gemisch aus einem Ethylen-Alpha-Olefin-Elastomer und einem Polyolefin-Copolymer umfassen, das mit Maleinsäureanhydrid gepfropft ist.

4. Mehrschicht-Bioreaktorbeutel nach einem der vorhergehenden Ansprüche, wobei die Mehrschichtfolie sechs Schichten (Schichten 1 bis 6) in serieller Reihenfolge umfasst, wie unten aufgeführt:
Schicht 1: die innere externe Oberflächenschicht (11);
Schicht 2: eine erste Ethylen-Alpha-Olefin-Elastomer-Strukturschicht (12);
Schicht 3: eine erste Verbindungsschicht (13);
Schicht 4: die Gassperrschicht (14);
Schicht 5: eine zweite Verbindungsschicht (15); und
Schicht 6: die äußere externe Oberflächenschicht (16);
wobei die sechs Schichten optional folgende Dickenbereiche aufweisen:
Schicht 1 liegt in einem Dickenbereich von 10-100 Mikrometern;
Schicht 2 liegt in einem Dickenbereich von 100-250 Mikrometern;
Schicht 3 liegt in einem Dickenbereich von 5-50 Mikrometern;
Schicht 4 liegt in einem Dickenbereich von 5-50 Mikrometern;
Schicht 5 liegt in einem Dickenbereich von 5-50 Mikrometern; und
Schicht 6 liegt in einem Dickenbereich von 10-100 Mikrometern;
wobei die sechs Schichten ferner optional folgende Dichtebereiche aufweisen:
Schicht 1 liegt in einem Dichtebereich von 910 bis 940 kg/m3 (0,91-0,94 g/cm³);
Schicht 2 liegt in einem Dichtebereich von 870 bis 910 kg/m3 (0,87-0,91 g/cm³);
Schicht 3 liegt in einem Dichtebereich von 870 bis 910 kg/m3 (0,87-0,91 g/cm³);
Schicht 4 liegt in einem Dichtebereich von 110 bis 125 kg/m3 (1,10-1,25 g/cm³);
Schicht 5 liegt in einem Dichtebereich von 870 bis 910 kg/m3 (0,87-0,91 g/cm³); und
Schicht 6 liegt in einem Dichtebereich von 910 bis 940 kg/m3 (0,91-0,94 g/cm3).

5. Mehrschichtiger Bioreaktorbeutel nach einem der vorhergehenden Ansprüche, wobei die Gassperrschicht (14) EVOH ist.

6. Mehrschichtiger Bioreaktorbeutel nach Anspruch 1, umfassend einen aufblasbaren Beutel (20) mit einer geschlossenen zentralen Kammer (21) zum Halten einer Zelle oder eines flüssigen Bioreaktormediums, wobei der aufblasbare Beutel (20) ein oberes Blatt (22) aus mehrschichtigem Polymermaterial und ein untere Blatt (24) aus mehrschichtigem Polymermaterial umfasst, wobei das mehrschichtige Polymermaterial die Mehrschichtfolie (10) umfasst.

7. Mehrschichtiger Bioreaktorbeutel nach Anspruch 6, wobei das obere Blatt (22) und das untere Blatt (24) entlang der Umfangskanten heißversiegelt sind, um die zentrale Kammer (21) zu bilden, wobei gegebenenfalls eine Grifföffnung (25) in einer oberen Umfangskante des Beutels (20) vorgesehen ist; und wobei Anschlussöffnungen für Aufnahmeröhrchen (27) in einer unteren Umfangskante des Beutels (20) vorgesehen sind.

8. Mehrschicht-Bioreaktorbeutel nach einem der vorhergehenden Ansprüche 1 bis 7, wobei die internen Schichten eines oder mehr aus anhydridmodifiziertem Polyethylen, Ethylen-/ungesättigtem Säurecopolymer, Ethylen-/ungesättigtem Estercopolymer, anhydridmodifiziertem Polyolefin, Polyurethan und Mischungen daraus umfassen.

9. Mehrschichtiger Bioreaktorbeutel nach Anspruch 1, umfassend einen Einweg-Bioprozessbeutel für die Züchtung von Zellmedien, wobei
das Ethylen-Alpha-Olefin-Elastomer ein Octen-1-Elastomer auf Ethylenbasis ist; und
die Gassperrschicht ein Ethylenvinylalkohol-Copolymer ist.

10. Mehrschicht-Bioreaktorbeutel nach einem der vorhergehenden Ansprüche 1 bis 9, wobei das Verhältnis der Schichtdicke jeder der externen Oberflächenschichten (11, 16) zur Schichtdicke der Strukturschicht (12) zwischen 1:2,5 und 1:10 liegt.

11. Mehrschichtfolie (10), umfassend:
innere und äußere externe Oberflächenschichten (11, 16) auf gegenüberliegenden Seiten der Mehrschichtfolie (10), die im Wesentlichen aus einem Polyethylen niedriger Dichte mit hohem Reinheitsgrad und ohne Zusatzstoffe bestehen und eine Dichte in einem Bereich von 910 bis 940 kg/m3 (0,91 bis 0,94 g/cm³) aufweisen, um nichtklebrige äußere Oberflächenschichten zu erzeugen;
interne Schichten, die zwischen den externen Oberflächenschichten angeordnet sind, die eine Gassperrschicht (14) und eine Strukturschicht (12) umfassen, die ein Ethylen-Alpha-Olefin-Elastomer mit einer Dichte in einem Bereich von 870 bis 910 kg/m3 (0,87-0,91 g/cm³) umfasst, um einen flexiblen Träger für die Mehrschichtfolie bereitzustellen; und
eine oder mehrere Verbindungsschichten (13, 15), um die internen Schichten mit einer oder mehreren der Ethylen-Alpha-Olefin-Elastomer-Strukturschichten (12) und der Oberflächenschichten (11, 16) zu verbinden.

12. Mehrschichtfolie nach Anspruch 11, wobei die eine oder mehrere Verbindungsschichten (13, 15) ein Gemisch aus einem Ethylen-Alpha-Olefin-Elastomer und einem Polyolefin-Copolymer umfassen, das mit Maleinsäureanhydrid gepfropft ist.

13. Mehrschichtfolie nach Anspruch 11 oder Anspruch 12, wobei die Mehrschichtfolie sechs Schichten (Schichten 1 bis 6) in serieller Reihenfolge umfasst, wie unten aufgeführt:
Schicht 1: die innere äußere Oberflächenschicht (11);
Schicht 2: eine erste Ethylen-Alpha-Olefin-Elastomer-Strukturschicht (12);
Schicht 3: eine erste Verbindungsschicht (13);
Schicht 4: die Gassperrschicht (14);
Schicht 5: eine zweite Verbindungsschicht (15); und
Schicht 6: die äußere externe Oberflächenschicht (16);
wobei die sechs Schichten optional folgende Dickenbereiche aufweisen:
Schicht 1 liegt in einem Dickenbereich von 10-100 Mikrometern;
Schicht 2 liegt in einem Dickenbereich von 100-250 Mikrometern;
Schicht 3 liegt in einem Dickenbereich von 5-50 Mikrometern;
Schicht 4 liegt in einem Dickenbereich von 5-50 Mikrometern;
Schicht 5 liegt in einem Dickenbereich von 5-50 Mikrometern; und
Schicht 6 liegt in einem Dickenbereich von 10-100 Mikrometern;
wobei die sechs Schichten ferner optional die folgenden Dichtebereiche aufweisen:
Schicht 1 liegt in einem Dichtebereich von 910 bis 940 kg/m3 (0,91-0,94 g/cm³);
Schicht 2 liegt in einem Dichtebereich von 870 bis 910 kg/m3 (0,87-0,91 g/cm³);
Schicht 3 liegt in einem Dichtebereich von 870 bis 910 kg/m3 (0,87-0,91 g/cm³);
Schicht 4 liegt in einem Dichtebereich von 110 bis 125 kg/m3 (1,10-1,25 g/cm³);
Schicht 5 liegt in einem Dichtebereich von 870 bis 910 kg/m3 (0,87-0,91 g/cm³); und
Schicht 6 liegt in einem Dichtebereich von 910 bis 940 kg/m3 (0,91-0,94 g/cm³).

14. Mehrschichtfolie nach einem der vorhergehenden Ansprüche 11 bis 13, wobei die Gassperrschicht (14) EVOH ist.

15. Mehrschichtfolie nach einem der vorhergehenden Ansprüche 11 bis 14, wobei die internen Schichten eines oder mehr aus anhydridmodifiziertem Polyethylen-, Ethylen-/ungesättigtem Säure-Copolymer, Ethylen-/ungesättigtem Ester-Copolymer, anhydridmodifiziertem Polyolefin, Polyurethan und Mischungen daraus umfassen.

## Revendications

1. Sac de bioréacteur multicouche (20) fabriqué à partir d'un film multicouche (10), comprenant :
des couches de surface extérieures interne et externe (11, 16) sur des faces opposées du film multicouche (10) constituées essentiellement d'un polyéthylène basse densité avec un niveau élevé de pureté, sans additifs, et avec une densité comprise entre 910 et 940 kg/m³ (0,91 et 0,94 g/cc), afin de fournir des couches de surface extérieures non collantes ;
des couches intérieures disposées entre les couches de surface extérieures et comprenant une couche barrière aux gaz (14) et une couche structurelle (12) qui comprend un élastomère d'éthylène et d'alpha-oléfine ayant une densité comprise entre 870 et 910 kg/m³ (0,87 et 0,91 g/cc), afin de fournir un support flexible au film multicouche.

2. Sac de bioréacteur multicouche selon la revendication 1, dans lequel le film multicouche comprend en outre une ou plusieurs couches de liaison (13, 15) afin de lier les couches intérieures à une ou plusieurs parmi la couche structurelle d'élastomère d'éthylène et d'alphaoléfine (12) et les couches de surface (11, 16).

3. Sac de bioréacteur multicouche selon la revendication 2, dans lequel la ou les couches de liaison (13, 15) comprennent un mélange d'un élastomère d'éthylène et d'alpha-oléfine et d'un copolymère de polyoléfine greffé avec de l'anhydride maléique.

4. Sac de bioréacteur multicouche selon l'une quelconque des revendications précédentes, dans lequel le film multicouche comprend six couches (couches 1 à 6) listées ci-dessous dans un ordre croissant :
couche 1 : la couche de surface extérieure interne (11) ;
couche 2 : une première couche structurelle d'élastomère d'éthylène et d'alpha-oléfine (12) ;
couche 3 : une première couche de liaison (13) ;
couche 4 : la couche barrière aux gaz (14) ;
couche 5 : une deuxième couche de liaison (15) ; et
couche 6 : la couche de surface extérieure externe (16),
éventuellement dans lequel les plages d'épaisseur des six couches sont les suivantes :
la couche 1 a une épaisseur comprise entre 10 et 100 microns ;
la couche 2 a une épaisseur comprise entre 100 et 250 microns ;
la couche 3 a une épaisseur comprise entre 5 et 50 microns ;
la couche 4 a une épaisseur comprise entre 5 et 50 microns ;
la couche 5 a une épaisseur comprise entre 5 et 50 microns ; et
la couche 6 a une épaisseur comprise entre 10 et 100 microns,
en outre éventuellement dans lequel les plages de densité des six couches sont les suivantes :
la couche 1 a une densité comprise entre 910 et 940 kg/m³ (0,91 et 0,94 g/cc) ;
la couche 2 a une densité comprise entre 870 et 910 kg/m³ (0,87 et 0,91 g/cc) ;
la couche 3 a une densité comprise entre 870 et 910 kg/m³ (0,87 et 0,91 g/cc) ;
la couche 4 a une densité comprise entre 110 et 125 kg/m³ (1,10 et 1,25 g/cc) ;
la couche 5 a une densité comprise entre 870 et 910 kg/m³ (0,87 et 0,91 g/cc) ; et
la couche 6 a une densité comprise entre 910 et 940 kg/m³ (0,91 et 0,94 g/cc).

5. Sac de bioréacteur multicouche selon l'une quelconque des revendications précédentes, dans lequel la couche barrière aux gaz (14) est une couche EVOH.

6. Sac de bioréacteur multicouche selon la revendication 1, comprenant un sac gonflable (20) ayant une chambre centrale fermée (21) destinée à contenir un milieu liquide de cellule ou de bioréacteur, le sac gonflable (20) comprenant une feuille supérieure (22) de matériau polymère multicouche et une feuille inférieure (24) de matériau polymère multicouche, dans lequel le matériau polymère multicouche comprend le film multicouche (10).

7. Sac de bioréacteur multicouche selon la revendication 6, dans lequel la feuille supérieure (22) et la feuille inférieure (24) sont thermoscellées le long de bords périmétriques afin de former la chambre centrale (21), éventuellement dans lequel une ouverture de poignée (25) est prévue dans un bord périmétrique supérieur du sac (20), et des ouvertures d'orifice destinées à recevoir des tubes (27) sont prévues dans un bord périmétrique inférieur du sac (20).

8. Sac de bioréacteur multicouche selon l'une quelconque des revendications 1 à 7, dans lequel les couches intérieures comprennent un ou plusieurs parmi un polyéthylène modifié par anhydride, un copolymère d'éthylène et d'acide insaturé, un copolymère d'éthylène et d'ester insaturé, une polyoléfine modifiée par anhydride, un polyuréthane, et des mélanges de ceux-ci.

9. Sac de bioréacteur multicouche selon la revendication 1, comprenant un sac de biotraitement à usage unique destiné à un milieu cellulaire de croissance, dans lequel :
l'élastomère d'éthylène et d'alpha-oléfine est un élastomère octène-1 à base d'éthylène ; et
la couche barrière aux gaz est un copolymère d'éthylène et d'alcool vinylique.

10. Sac de bioréacteur multicouche selon l'une quelconque des revendications 1 à 9, dans lequel le rapport de l'épaisseur de couche de chacune des couches de surface extérieures (11, 16) à l'épaisseur de couche de la couche structurelle (12) est compris entre 1:2,5 et 1:10.

11. Film multicouche (10), comprenant :
des couches de surface extérieures interne et externe (11, 16) sur des faces opposées du film multicouche (10) constituées essentiellement d'un polyéthylène basse densité avec un niveau élevé de pureté, sans additifs, et avec une densité comprise entre 910 et 940 kg/m³ (0,91 et 0,94 g/cc), afin de fournir des couches de surface extérieures non collantes ;
des couches intérieures disposées entre les couches de surface extérieures et comprenant une couche barrière aux gaz (14) et une couche structurelle (12) qui comprend un élastomère d'éthylène et d'alpha-oléfine avec une densité comprise entre 870 et 910 kg/m³ (0,87 et 0,91 g/cc), afin de fournir un support flexible au film multicouche ; et
une ou plusieurs couches de liaison (13, 15) afin de lier les couches intérieures à une ou plusieurs parmi la couche structurelle d'élastomère d'éthylène et d'alpha-oléfine (12) et les couches de surface (11, 16).

12. Film multicouche selon la revendication 11, dans lequel la ou les couches de liaison (13, 15) comprennent un mélange d'un élastomère d'éthylène et d'alpha-oléfine et d'un copolymère de polyoléfine greffé avec de l'anhydride maléique.

13. Film multicouche selon la revendication 11 ou 12, dans lequel le film multicouche comprend six couches (couches 1 à 6) listées ci-dessous dans un ordre croissant :
couche 1 : la couche de surface extérieure interne (11) ;
couche 2 : une première couche structurelle d'élastomère d'éthylène et d'alpha-oléfine (12) ;
couche 3 : une première couche de liaison (13) ;
couche 4 : la couche barrière aux gaz (14) ;
couche 5 : une deuxième couche de liaison (15) ; et
couche 6 : la couche de surface extérieure externe (16),
éventuellement dans lequel les plages d'épaisseur des six couches sont les suivantes :
la couche 1 a une épaisseur comprise entre 10 et 100 microns ;
la couche 2 a une épaisseur comprise entre 100 et 250 microns ;
la couche 3 a une épaisseur comprise entre 5 et 50 microns ;
la couche 4 a une épaisseur comprise entre 5 et 50 microns ;
la couche 5 a une épaisseur comprise entre 5 et 50 microns ; et
la couche 6 a une épaisseur comprise entre 10 et 100 microns,
en outre éventuellement dans lequel les plages de densité des six couches sont les suivantes :
la couche 1 a une densité comprise entre 910 et 940 kg/m³ (0,91 et 0,94 g/cc) ;
la couche 2 a une densité comprise entre 870 et 910 kg/m³ (0,87 et 0,91 g/cc) ;
la couche 3 a une densité comprise entre 870 et 910 kg/m³ (0,87 et 0,91 g/cc) ;
la couche 4 a une densité comprise entre 110 et 125 kg/m³ (1,10 et 1,25 g/cc) ;
la couche 5 a une densité comprise entre 870 et 910 kg/m³ (0,87 et 0,91 g/cc) ;
la couche 6 a une densité comprise entre 910 et 940 kg/m³ (0,91 et 0,94 g/cc).

14. Film multicouche selon l'une quelconque des revendications 11 à 13, dans lequel la couche barrière aux gaz (14) est une couche EVOH.

15. Film multicouche selon l'une quelconque des revendications 11 à 14, dans lequel les couches intérieures comprennent un ou plusieurs parmi un polyéthylène modifié par anhydride, un copolymère d'éthylène et d'acide insaturé, un copolymère d'éthylène et d'ester insaturé, une polyoléfine modifiée par anhydride, un polyuréthane, et des mélanges de ceux-ci.
